(19) Europäisches Patentamt
European Patent Office
Office européen des brevets

(11) **EP 3 639 745 B1**

(12) **EUROPEAN PATENT SPECIFICATION**

(45) Date of publication and mention
of the grant of the patent:
**15.03.2023 Bulletin 2023/11**

(21) Application number: **17772081.0**

(22) Date of filing: **15.06.2017**

(51) International Patent Classification (IPC):
*A61B 6/00* (2006.01)     *A61B 8/00* (2006.01)
*G01T 1/00* (2006.01)

(52) Cooperative Patent Classification (CPC):
**A61B 6/4405; A61B 6/4258; A61B 6/4417;
A61B 6/5205; A61B 8/00; G01T 1/1603;
G01T 1/161**

(86) International application number:
**PCT/ES2017/070435**

(87) International publication number:
**WO 2018/229311 (20.12.2018 Gazette 2018/51)**

(54) **DUAL IMAGE SYSTEM SUITABLE FOR ONCOLOGICAL DIAGNOSES AND REAL TIME GUIDED BIOPSIES**

DUALES BILDGEBUNGSSYSTEM FÜR ONKOLOGISCHE DIAGNOSEN UND ECHTZEIT-GEFÜHRTE BIOPSIEN

SYSTÈME D'IMAGE DUALE CONÇU POUR SON UTILISATION DANS DES DIAGNOSTICS ONCOLOGIQUES ET BIOPSIES GUIDÉES EN TEMPS RÉEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(43) Date of publication of application:
**22.04.2020 Bulletin 2020/17**

(73) Proprietors:
• **Consejo Superior de Investigaciones Científicas (CSIC)**
**28006 Madrid (ES)**
• **Universitat de València**
**46010 Valencia (ES)**

(72) Inventors:
• **CABALLERO ONTANAYA, Luis**
**46980 Paterna - Valencia (ES)**
• **DOMINGO PARDO, César**
**46980 Paterna - Valencia (ES)**
• **ALBIOL COLOMER, Francisco Javier**
**46980 Paterna - Valencia (ES)**

(74) Representative: **Temiño Ceniceros, Ignacio**
**Abril Patentes y Marcas, S.L.**
**Calle Zurbano, 76 - 7°**
**28010 Madrid (ES)**

(56) References cited:
**JP-A- 2005 013 291     US-A1- 2004 054 248
US-A1- 2013 237 811**

• **AYA KISHIMOTO ET AL: "First demonstration of multi-color 3-D in vivo imaging using ultra-compact Compton camera", SCIENTIFIC REPORTS, vol. 7, no. 1, 18 May 2017 (2017-05-18), XP055457113, DOI: 10.1038/s41598-017-02377-w**

**Description**

**FIELD OF THE INVENTION**

The present invention is reported within the technical field of the oncological surgery, and more specifically, to the image technologies, diagnosis and treatment of tumor diseases. The invention relates without limitation to the development of a portable and light dual h gamma rays and ultrasound imaging system, suitable for medical use in oncological diagnoses, which allows to perform a guided biopsy, providing a functional and anatomic real time visualization of the tumor zone, while tissue extraction is performed. Said system comprises an ultrasonic camera, and a gamma radiation camera based on Compton dispersion of the gamma rays.

**BACKGROUND OF THE INVENTION**

In the past, diagnoses, detection and treatments of tumors have typically been based on nuclear medicine technologies and methods, such Positron Emission Tomography (PET) or Single Photon Emission Computed Tomography (SPECT) or planar gammagraphy. These technologies, based on the gamma ray emission, allow to obtain images of the patient body based on the radiation produced after the injection of radioactive substances associated to molecules with large tumor affinity; those substances emit gamma rays through disintegration and after their detection, it is possible to reconstruct the origin of the radiation and identify the area susceptible to cancer for its further treatment. These technologies allow, therefore, a functional analysis of the area of interest, although they present certain limitations in terms of resolution and level of access to certain remote areas of the human body, since they require large size equipment in order to obtain the adequate accuracy, mainly due to the use of a collimator between its components.

Furthermore, a biopsy is necessary for the effective cancer diagnosis and to remove false positives; that is, to distinguish between cancerous and non cancerous cells. The kind of image that typically guides biopsies is a morphological one: mammographies, magnetic resonances or ultrasound, being this last technique the one used for needle guide during suspicious tissue extraction. Nevertheless, a combination with imaging techniques that offer functional information for a broadening of specification and diagnosis sensitivity is required. With that purpose, ultrasonic cameras (which provide anatomical information) have been combined with gamma ray detection cameras (that provide functional information) in the past, in order to increase the efficiency on the detection of tumor zones. Recently, combined ultrasound and gamma rays imaging systems from the tumor zones has demonstrated to be useful for conducting guided biopsies more effectively; meaning, extracting tissues from the suspicious area while taking images. However, in some cases the system size hinders or obstructs access of the needle itself to the biopsy. In other cases, the system size prevents its use in some areas of human bodies. So, even with the existence of these systems, there is a need for a lightweight and handheld instrument able to provide real time dual images, allowing the diagnose anywhere in the patient's body and the performance of guided biopsies with a high accuracy, this being a critical factor considering the demonstrated importance of tumor heterogeneity to determine the most adequate treatment for the patient and the future response of the tumor.

Document JP 2005 013291 A (Hitachi Medical Corp) discloses a dual imaging system comprising an ultrasound imaging device and a gamma ray detector, an electronic module and a casing that houses both detectors. However, its design is not compact, thus requiring a system of substantial space in order to improve the localization of the source of radiation in the patient's body.

In summary, with the aforementioned limitations, it is necessary, in the present technical field, to provide a dual image system that:

- allows to obtain functional information about the patient's body with a high accuracy and with low radiation dose;
- allows to obtain anatomical information from the patient's body;
- has a reduced size that makes it portable and handheld;
- allows to control and transmit the real time acquired information;
- possesses flexibility for its use in every part of the patient's body;
- allows the use of a needle to perform precise biopsies simultaneously with the imaging.

The present invention provides a novel imaging system, devised for conducting guided biopsies through real time dual images, created with ultrasonic and gamma radiation Compton technique cameras, whose efficiency improves known solutions of the state of the art, and whose performance allows to overcome with the same device the problems detailed before. Furthermore, the invention is oriented to provide a reduced and compact system, facilitating the deployment of the cameras over the patient, regardless of the remote access to the area near the tumor area. At the same time, the invention is focused to facilitate the manual handling by the specialized personnel and its transport.

## SUMMARY OF THE INVENTION

A main object of the present invention relates without limitation to the development of a dual imaging system, suitable for use in oncological diagnoses and real time guided biopsies that comprises, preferably:

- an ultrasound imaging device for obtaining associated signals to one or more anatomical images;
- a gamma ray detector for obtaining associated signals to one or more functional images;
- an electronic module for processing the obtained signals by the ultrasound imaging device and the gamma ray detector;
- a casing that houses the ultrasound imaging device and the gamma ray detector.

Advantageously, in said system, the gamma ray Compton detector is a Compton gamma ray scatter detector, that typically comprises at least two gamma detection regions substantially aligned in the same detection direction and capable of obtaining the position and energy of the detected gamma rays in each of the regions. Likewise, the ultrasound imaging device and the gamma ray detector possess detection directions substantially parallel with each other, making preferably an angle between 0-90 degrees, where the position corresponding to 0 degrees corresponds to a parallel alignment between the two.

In that way, it is achieved to remove the need of a collimator in order to obtain an adequate resolution in the functional images. The fact that no collimator is used in the system of the present invention and that said system is based on a Compton reconstruction does not limit the range of energy of the incident radiation and, above all, it makes that the detection system is not designed exclusively for a particular energy. That is, gamma ray detectors that employ a collimator limit the system capabilities in terms of optimum energy range for the image reconstruction and, above all, they substantially reduce detection sensitivity of the system (considering only the detected events of gamma rays that must necessarily have passed through the collimator) and also make the equipment very heavy and difficult to operate. In the case of the present invention, a mechanical collimator is not necessary because the reconstruction of the image occurs from coincident events between both detectors. In such way, image resolution is improved and that allows the performance of a guided biopsy with higher efficiency.

In another preferred embodiment of the invention, in the dual imaging system each gamma detection region comprises a plurality of scintillation crystals coupled to a solid state photosensor and to semiconductor-type detectors, for the gamma radiation detection and a plurality of photomultipliers to form an electric pulse that can be processed to amplify the detected signal by said scintillation crystals.

Preferably, the scintillation crystals are monolithic or pixelated scintillation crystals, such as: NaI, CsI, LaBr$_3$, LaCl$_3$ or LYSO, said scintillation crystals being coupled to sensitive photomultipliers to the position. More preferably, the photomultipliers are of semiconductor type. Likewise, gamma ray detectors can consist of semiconductor detectors such as HPGe or CdZnTe.

This results in high compactness, due to the use of solid state semiconductors such as photomultipliers in radiation detectors.

Even more preferably, the invention system comprises silicon photomultipliers of the Silicon Photomultipliers (SiPMs) type, that offers advantages in terms of speed, noise-signal ratio, consumption and size, since they are really compact. The SiPMs photomultipliers are coupled to scintillation crystals, which are ideally suitable for the utilization of the generated information by the Compton gamma ray scattering phenomenon; specially, for the coupling with an ultrasound transducer, since the system must be generally light to be handheld by the medical personnel. The use of silicon photosensors allows the design of a low-power, low-amperage and low-voltage (tens of volts vs. hundreds of volts that conventional photomultiplier tubes require) device, which is an advantage for its intraoperative use.

In another preferred embodiment of the invention, the system comprises a computational module connected to an electronic module, with a data visualization display for showing anatomic and functional images.

In another preferred embodiment of the invention, the system comprises a needle to perform guided biopsies.

In that way, it is achieved the performance of guided biopsies with a high accuracy because of the combination of morphological and functional information, being this last factor critical considering the importance of the tumor heterogeneity to determine the patient's treatment and the future tumor response. Reconstruction of images with a large amount of information in real time is also achieved while performing a biopsy.

In another preferred embodiment of the invention, the system comprises two or more Compton detectors.

In that sense, it is possible to reconstruct with higher accuracy the distribution of the radioactive tracers for its subsequent combination with the image obtained by ultrasound detectors. This second Compton detector clarifies the ambiguities in the reconstruction of the image. Preferably this second Compton detector is symmetrical to the first Compton detector and is located on one side of the ultrasound imaging device.

In another preferred embodiment of the invention, the Compton detector/s is/are disposed in juxtaposition to the ultrasound imaging device on the sides of said ultrasound imaging device.

In another preferred embodiment of the invention, the ultrasound imaging device is removable for its use independent from the Compton detector/s. More preferably, the Compton detector/s can be coupled to a standard external ultrasound imaging device.

It is achieved in this way the use of the ultrasound imaging device if it shall be useful or necessary for other diagnostics or uses where the gamma ray detector is not required.

In another preferred embodiment of the invention, the data transmission between the Compton detectors, the electronic module and the computational module is done by wiring or by wireless communication.

In another preferred embodiment of the invention, the system further comprises a collimator coupled to a Compton detector.

It is possible, in this way, to optionally add a mechanic collimator, for example, for the first individual front gamma ray dispersion detector (such as parallel hollow collimator fan-beam collimators, "coded masks", etc.) that could facilitate a better determination of the incident position in any particular case.

In another preferred embodiment of the invention, the distance and angle between each individual gamma ray Compton detector may be fixed or be mechanical adjustable. Alternatively, it is possible to use a monolithic detector of a certain volume, capable of distinguish al least two interactions of a gamma ray inside said volume.

Thus, it is possible, to adjust the number of events measured and the uncertainty in the detection, as needed from the information provided by the ultrasound image to maximize the result based on the damage position.

In another preferred embodiment of the invention, at least one Compton detector comprises three or more individual gamma detectors, substantially aligned in the same detection direction and with detection surfaces substantially parallel between them. Thus, it is achieved to provide a higher detection efficiency, which improves the event statistics, and, hence, reduces the time needed for reconstructing the functional image of the patient's tissue.

Another object of the invention is related to a method of obtaining anatomical and functional dual images of a patient that comprises the use of a dual image system according to the previous embodiments and carrying out the following steps:

- the system is deployed on a surface or body;
- anatomical information of said surface or body is generated by the ultrasound imaging device;
- functional information of said surface or body is generated by the Compton detector/s;
- the signals obtained by the ultrasound imaging device and Compton detectors are processed by the electronic module;
- the processed signals are sent to the computational module;
- the anatomical and functional images are shown in real time on a display from the computational module.

In addition, in other preferred embodiments of the invention, dual images obtained through the aforementioned method can be combined with other images or data obtained during previous or posterior stages, providing a larger combined information.

Thus, the invention allows to propose a solution to the technical problems described in previous sections, and provides a dual imaging system that facilitates obtaining real time functional and anatomical dual images with high accuracy, overcoming the technical features described in the documents of the state of the art. The invention is suitable for its use in guided biopsies due to its compactness, easy operation, portability and wide measurement range, which makes it especially useful for oncological diagnosis and treatments.

## DESCRIPTION OF THE DRAWINGS

Figure 1 shows a perspective view of the detectors that comprises the dual image system, according to a preferred embodiment of the invention.

Figure 2 shows a detailed scheme of a Compton detector of the system, according to a preferred embodiment of the invention.

Figure 3 shows a side view of the detectors of the system, according to a preferred embodiment of the invention.

Figure 4 shows a scheme of the main elements of the system, according to a preferred embodiment of the invention.

Figures 5A-5B show an example of an anatomical ultrasound image obtained through conventional techniques (5A) and a functional and anatomical image obtained through the system (5B), according to a preferred embodiment of the invention.

## NUMERICAL REFERENCES USED IN THE DRAWINGS

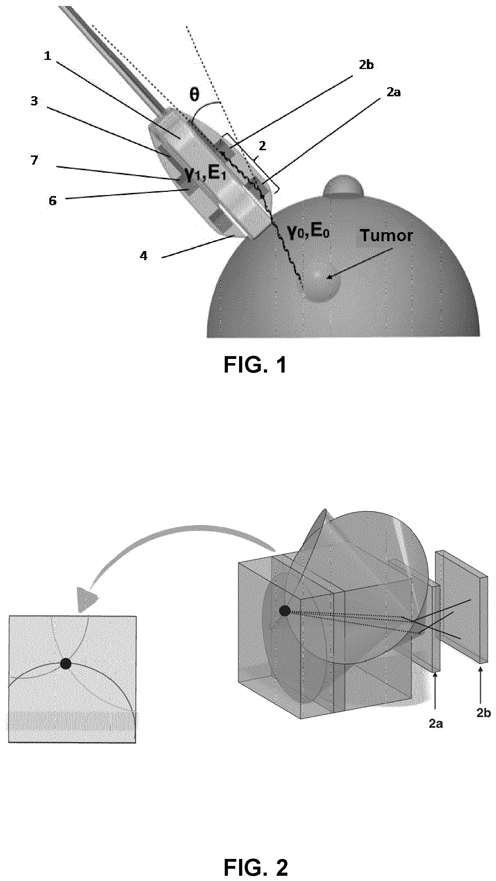

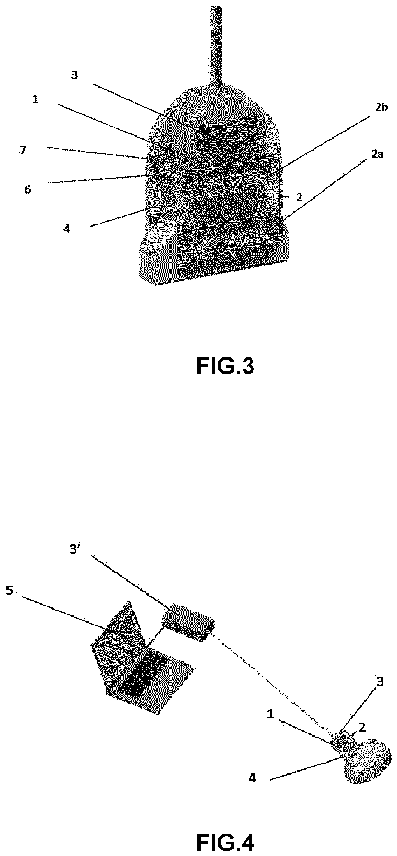

In order to provide a better understanding of the technical features of the invention, the referred Figures 1-5 are accompanied of a series of numeral references which, with illustrative and non limiting character, are hereby represented:

| (1) | Ultrasound imaging device |
| --- | --- |
| (2) | Gamma ray Compton detector |
| (2a) | Individual front gamma ray scattering detector |
| (2b) | Individual back gamma ray absorption detector |
| (3) | Electronic module |
| (3') | Signal processing module |
| (4) | Casing |
| (5) | Computational module |
| (6) | Scintillation crystal |
| (7) | Photomultiplier |

## DETAILED DESCRIPTION OF THE INVENTION

An exemplary preferred embodiment of the present invention is described for purposes of illustration, but not limitation thereof, will now be described.

As described in the previous sections, a principal object of the invention is related to a dual imaging system, suitable for the performance of guided biopsies through images based on ultrasound imaging for the providing of signals associated to one or more anatomical images and gamma radiation device for oncological diagnostics.

In a preferred embodiment of the invention, as shown in the Figures 1-5, the dual imaging system comprises an ultrasound imaging device (1), at least one gamma radiation Compton detector (2), an electronic module (3), a signal processing module (3'), a casing (4) and a computational module (5).

Preferably, each gamma radiation Compton detector (2), as shown in the Figure 2, comprises at least two gamma detection regions, substantially aligned in the same detection direction. These two gamma detection regions can be two individual gamma radiation detectors (2a, 2b), defining two detection planes, able to measure interaction and energy positions. Alternatively, it is possible to use a monolithic detector of a certain volume capable of distinguish at least two interactions of a gamma ray inside said volume. More preferably, as in Figures 1-5, each Compton detector (2) has an individual front gamma ray scattering detector (2a) and an individual back gamma ray absorption detector (2b). Optionally, the frontal detector (2a) and the back absorption detector (2b) can exchange their functionality, depending on the direction of the gamma radiation.

An imaging formation device using a Compton detector (2) is based on the detection of gamma rays that have interacted one or more times in the back absorption detector (2b) after those gamma rays, proceeding from a gamma ray source, have been Compton scattered by a front scattering detector (2a). The events are registered in a narrow time coincidence window to ensure the belonging to a single gamma ray incident. The spatial distribution of the gamma ray source is reproduced based on the interaction of information (that is, the detected position and energy of the gamma ray in each detector (2a, 2b)).

When at least the position and lost energy of the initial gamma ray in the scattering detector (2a) it is known and also the position of the gamma ray that interacts in the absorption detector (2b) is know, and providing that both events take place inside the defined time coincidence window, it is possible to calculate the original path or, at least, the cone of possible paths. By accumulating different trajectory cones (see Fig. 2), the emitter source of radiation position from the patient's body can be reconstructed.

As shown in Figure 2, a Compton detector requires to distinguish between the first gamma ray interaction (its position and energy) relative to the second interaction position. For that purpose, it is convenient a system with at least two detection surfaces, although it is possible to use a single detector, sensitive to multiple interactions, as to mount more detection surfaces. In that last case, detection surfaces may be separated by a suitable distance in order to take sufficient statistics of events to reconstruct the final gamma image with known accuracy.

Both the front scattering detector (2a) and the back absorption detector (2b) preferably comprise a scintillation crystal (6) coupled to a solid state photomultiplier (7), more preferably, made of silicon, and also comprise semiconductor detectors.

Compactness is achieved thanks to scintillation crystals (6) and solid state photomultipliers (7). Preferably, said photomultipliers (7) are made of silicon. On the one hand, the smaller size of said silicon photomultipliers (7) if compared to typical photomultiplier tubes (used in the prior art) and, on the other hand, the elimination of the need to introduce a collimator, confer to the invention said advantage, allowing the construction of Compton detectors and the dual imaging system with a reduced size.

More precisely, the elements for the performance of the dual imaging system are described below, according to this preferred embodiment of the invention, and as shown in Figures 1-5:

- an ultrasound imaging device (1) which allows obtaining anatomic images of the tissue and whose detector direction is preferably aligned to the detection direction of the plurality of gamma ray Compton detectors (2);
- a plurality of gamma ray Compton detectors (2), which allows obtaining images with functional information from the patient's tissue;
- an electronic module (3) preferably located near to the photomultipliers (7), in order to collect the electric pulses generated for its digitalization; it includes a logic programmable and /or configurable electronics of the time window of gamma events, detected in the gamma ray detectors (2);
- a signal processing module (3') for recording the digitalized signal and for sending it to the computational module (5);
- a casing (4) for protecting the detectors (1, 2) and the electronic module (3);
- a computational module (5) (for example, a computer) to display the final image combined with the information generated by the ultrasound (1) and gamma ray (2) detectors on a screen or display.

As described above, each Compton detector (2) comprises two gamma ray detectors (2a, 2b). One being a front scattering detector (2a), and the other being a back absorption detector (2b), arranged such that they are substantially aligned in the same detection direction and with parallel detection surfaces.

In this preferred embodiment of the invention, the gamma ray detectors (2a, 2b) are preferably arranged at one side of the ultrasound imaging device (1).

A description of the dual ultrasound and gamma ray imaging system corresponding to the previously described system is detailed as following:

The combined gamma ray and ultrasound system of the invention is lightweight and can be handled as a conventional ultrasound device.

The gamma ray detector (2) described herein is based on the Compton scattering law of gamma rays. The gamma rays generated by the radionuclide within the patient's tissue arrive at the system and have a scattering Compton interaction at a first front scattering detector (2a), losing some of its energy. Afterwards, the scattered gamma ray deposits a part or the rest of its energy on the second back absorption detector (2b). By measuring the position and the energy of the gamma ray deposited in the first detector (2a), and the position of the gamma ray scattered in the second detector (2b), it is possible to known a collection of possible paths of the initial gamma ray emitted from the radio tracer injected in the patient's body through the following expression:

$$E_1 = \frac{E_0}{1 + \left(\dfrac{E_0}{m_0 c^2}\right)\left(1 - \cos\theta\right)}$$

Being $E_0$ and $E_1$ the energy of the initial and scattered gamma rays, respectively, and $\theta$ the scattering angle with respect to the initial path, such as shown in Figures 1 and 2. After a measuring time, the system collects a series of independent events like this, providing sufficient statistical information to obtain a spatial distribution of the radio tracers around the Compton detector (2).

The individual gamma ray detectors (2a, 2b) comprise scintillation crystals (6); preferably, pixelated or monolithic scintillation crystals (such as: Nal, Csl, LaBr3, LaCl3, LYSO, etc.), coupled to a solid state photomultiplier (7) sensitive to the position, for example, made of silicon.

When a gamma ray strikes the scintillation crystal (6), scintillation light is collected on the photomultiplier (7) which generates a signal which is amplified and sent to the electronic module (3) for its digitalization. Each individual gamma detection system (2a, 2b) generates an electrical pulse signal proportional to the deposited energy of the gamma ray incident on the scintillation crystal (6).

The electronic module (3) (flexible or rigid) for the gamma detectors (2a, 2b) signal digitalization is located near the photomultipliers (7). Said electronic module (3) converts the analogical signal into a digitalized value that is sent to the signal processing module (3'). The signal processing module (3') contains lectures and electric supply. The signal processing module (3') records the digitalized pulses and sends the data to a computational module (5), for example, a computer, and powers the individual gamma detectors (2a, 2b). The signal processing module (3') also selects the events in a well defined time window.

The signals from the ultrasound imaging device (1) are also sent to the signal processing module (3') and/or to the computational module (5) to process and generate the anatomical image.

Alternatively, the signal processing module (3') may be integrated in the electronic module (3) or in the computational module (5).

The signals from the individual gamma ray detectors (2a, 2b) are digitalized and sent to the computational module (5) or display in which gamma images are reconstructed and correlated with ultrasound images and are shown to the physician.

Images are typically displayed in 2D, since standard ultrasound imaging devices provide anatomical images in 2D. However, if the ultrasound imaging device allows 3D images, they can be merged with the 3D images that allow the generation of the Compton detectors and display a final dual 3D image.

The use of collimators is not necessary in the present invention, since the system is based on the Compton scattering phenomenon of gamma rays between the individual gamma detectors (2a, 2b), which provides the ability to determine the incidence position over each individual gamma detector (2a, 2b).

Alternatively, the dual image system may comprise a mechanical collimator for the first front scattering detector (2a) in order to improve the determination of the position (such as parallel hollow collimator, fan-beam collimators, coded masks, etc.) that could help a better determination of the position in some particular case.

In another preferred embodiment of the invention, the sistem comprises a second Compton detector (2). The most precise distribution of the radio tracers for its later combination with the ultrasound image, is achieved through this second Compton detector (2), substantially symmetrical to the first Compton detector (2), located on one side of the ultrasound imaging device (1). This second Compton detector (2) clarifies the ambiguities in the image reconstruction.

More preferably, the system comprises two Compton detectors (2), located one on each side of the ultrasound imaging device (see Fig. 1).

In another preferred embodiment of the invention, all the Compton detectors (2) are disposed at one side of the ultrasound detector (1).

In another preferred embodiment of the invention, the system comprises for the same Compton detector (2) more than two individual detectors (2a, 2b), substantially aligned in the same detection direction and with the detection surfaces substantially parallel with each other. A greater number of gamma detectors (2a, 2b) improves the statistic of events, and, therefore, the spatial resolution when reconstructing the functional image from the patient's tissue.

In another preferred embodiment of the invention, the distance between each individual gamma detector (2a, 2b) of each Compton detector (2) may be fixed or be mechanically adjustable, according to the needs from the information provided by the ultrasound image in order to maximize the result according to the position of the damage.

In another preferred embodiment of the invention, the data transmission between the Compton detectors (2) and the signal processing module (3') and/or the electronic module (3) and the computational module (5) takes place by wiring or by wireless means.

In another preferred embodiment of the invention, data processing of the gamma ray/ultrasound, calculation of the position, temporal coincidence of events, reconstruction of the image and image merging is performed in the signal processing module (3') and/or in the computational module (5) and displayed on a screen or display.

In another preferred embodiment of the invention, the system has two different configurations:

- a first system configuration with the two gamma ray (2) detectors and the ultrasound (1) imaging device coupled and integrated inside the casing (4), which provides combined real time images in 2D or 3D;
- a second system configuration with the gamma ray detector (2) being decoupled with the ultrasound imaging device (1), this being detachable, which provides anatomical images, typically in 2D, that are merged with the functional images, in 2D or 3D, from the information collected by the gamma ray detector (2).

In another preferred embodiment of the invention, the gamma ray detector (2) is coupled to an external ultrasound imaging device (1) comprising its own screen or display. Preferably, the gamma image is shown on the screen related to the ultrasound imaging device (1), overlapped with the anatomical image, through the external image input to said ultrasound detector (1) and display.

In another preferred embodiment of the invention wherein the gamma ray detector (2) is coupled to an external ultrasound imaging device (1), the external information generated by the ultrasound imaging device (1) is emitted from said imaging device (1) and introduced into the signal processing module (3') of the system to be processed and merged with the gamma image.

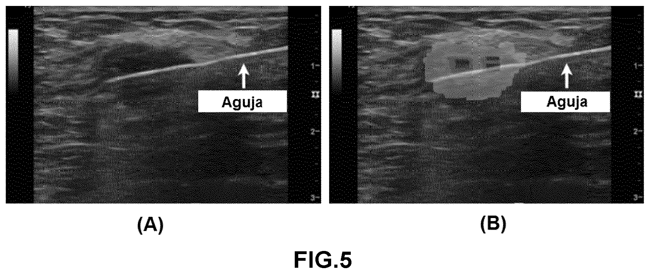

As an example, in Figures 5A-5B it is shown a comparison between the obtained results through a single anatomical ultrasound image (Fig. 5A) and a combination of an anatomical and functional image, obtained through the using of the system, according to a preferred embodiment of the invention (Fig. 5B). The obtained combined image provides detailed information about the collection of radioisotope in the patient's body. Said information can be shown with a color code that indicates the regions where tumor activity is large. This way, it is possible to perform a much more selective and precise biopsy in real time.

In this way, the present invention overcomes the technical issues described in previous sections and thus, provides a system which enables, preferably, the taking of combined anatomical and functional 2D and/or 3D images for real time diagnosis with a high resolution while performing a guided biopsy of tissue susceptible to be cancerous. In this context, the present invention allows obtaining metabolic information and a precise biopsy, being this a critical factor considering the demonstrated importance of the tumor heterogeneity to determine the most adequate patient treatment and the future response of the tumor.

On the other hand, the invention is aimed to provide a compact system, facilitating the deployment of the camera over the patient, regardless of the remote access to the area near the tumor. At the same time, the invention targets to facilitate manual handling by the specialized personnel and its transport.

## Claims

1. Dual image system, suitable for use in oncological diagnoses and real time guided biopsies, that comprises:

   - an ultrasound imaging device (1) for obtaining associated signals with one or more anatomical images;
   - a gamma ray detector (2) for obtaining associated signals with one or more functional images;
   - an electronic module (3) for processing signals obtained by the ultrasound imaging device (1) and by the gamma ray detector (2);
   - a casing (4) housing an ultrasound imaging device (1) and a gamma ray detector (2);

   and **characterized in that:**

   - the gamma ray detector (2) is a gamma ray Compton scattering detector, comprising at least two gamma ray detection regions, substantially aligned in the same detection direction;
   - the ultrasound imaging device (1) and the gamma ray detector (2) comprise detection surfaces forming an angle between 0 and 90 degrees;
   - the gamma ray detection regions of the Compton scattering detector comprise at least two individual gamma ray detectors (2a, 2b) equipped with a plurality of scintillation crystals (6) for the detection of gamma radiation, and a plurality of semiconductor photomultipliers (7) to amplify the signal detected by said scintillation crystals (6).

2. Dual image system, according to the preceding claim, wherein the scintillation crystals (6) comprises pixelated or monolithic scintillation crystal, such as: $LaBr_3$, $LaCl_3$ or LYSO; said scintillation crystals (6) being coupled to the photomultipliers (7).

3. Dual image system, according to claim 1, wherein the Compton detector (2) comprises a single detection volume, capable of distinguish two or more interactions of the same gamma ray inside said volume.

4. Dual image system, according to any one of the preceding claims, that further comprises a computational module (5) connected to an electronic module (3), with a screen or display in order to show anatomical and functional images.

5. Dual image system, according to any one of the preceding claims, that comprises two or more Compton detectors (2).

6. Dual image system, according to any one of the preceding claims, wherein the Compton detector/s (2) are disposed in juxtaposition to the ultrasound imaging device (1) at the sides of said ultrasound imaging device (1).

7. Dual image system, according to any one of the preceding claims, wherein the ultrasound imaging device (1) is detachable for its independent use from the Compton detector/s (2).

8. Dual image system, according to any of the preceding claims, wherein the data transmission between the Compton detector/s (2), the electronic module (3) and the computational module (5) is made by wiring or by wireless communication.

9. Dual image system, according to any of the preceding claims, that further comprises a collimator coupled to a Compton detector (2).

10. Dual image system, according to any of the preceding claims, wherein the relative distance and the relative angle between each gamma detection region of each Compton detector (2) may be mechanically adjustable.

11. Dual image system, according to any of the preceding claims, wherein at least one Compton detector (2) comprises two or more gamma ray detection regions, substantially aligned in the same detection direction and with detection surfaces substantially parallel to each other.

12. Dual image system, according to any of the preceding claims, that comprises a needle to perform guided biopsies.

13. Method for obtaining dual anatomical and functional images of a subject that comprises the use of a dual image system according to any of the preceding claims 1-12, and carrying out the following steps:

- the system is deployed on a surface or a subject's body;
- anatomical information of said surface or body is generated by the ultrasound imaging device (1);
- functional information of said surface or body is generated by the Compton detector/s (2);
- the signals obtained by the ultrasound (1) imaging device and Compton detector/s (2) are processed by the electronic module (3);
- the processed signals are sent to the computational module (5);
- the anatomical and functional images are shown in real time on a display from the computational module (5).


**Patentansprüche**

1. Duales Bildsystem, geeignet zur Verwendung bei onkologischen Diagnosen und in Echtzeit geführten Biopsien, das Folgendes umfasst:

- eine Ultraschallbildgebungsvorrichtung (1) zum Erhalten zugeordneter Signale mit einem oder mehreren anatomischen Bildern;
- einen Gammastrahlendetektor (2) zum Erhalten zugeordneter Signale mit einem oder mehreren funktionellen Bildern;
- ein elektronisches Modul (3) zum Verarbeiten von Signalen, die von der Ultraschallbildgebungsvorrichtung (1) und dem Gammastrahlendetektor (2) erhalten werden;
- ein Gehäuse (4), das eine Ultraschallabbildungsvorrichtung (1) und einen Gammastrahlendetektor (2) aufnimmt;

und **dadurch gekennzeichnet, dass:**

- der Gammastrahlendetektor (2) ein Gammastrahlen-Compton-Streudetektor ist, der mindestens zwei Gammastrahlen-Erfassungsbereiche umfasst, die im Wesentlichen in derselben Erfassungsrichtung ausgerichtet sind;
- die Ultraschallbildgebungsvorrichtung (1) und der Gammastrahlendetektor (2) Erfassungsoberflächen umfassen, die einen Winkel zwischen 0 und 90 Grad bilden;
- die Gammastrahlen-Erfassungsbereiche des Compton-Streudetektors mindestens zwei einzelne Gammastrahlendetektoren (2a, 2b), die mit mehreren Szintillationskristallen (6) zum Erfassen von Gammastrahlung ausgestattet sind, und mehrere Halbleiter-Photomultiplier (7) zum Verstärken des von den Szintillationskristallen (6) erfassten Signals umfassen.

2. Duales Bildsystem nach dem vorhergehenden Anspruch, wobei die Szintillationskristalle (6) pixelierte oder monolithische Szintillationskristalle umfassen, wie etwa: $LaBr_3$, $LaCl_3$ oder LYSO; wobei die Szintillationskristalle (6) mit den Photomultipliern (7) gekoppelt sind.

3. Duales Bildsystem nach Anspruch 1, wobei der Compton-Detektor (2) ein einziges Erfassungsvolumen umfasst, das in der Lage ist, zwei oder mehr Wechselwirkungen desselben Gammastrahls innerhalb des Volumens zu unterscheiden.

**4.** Duales Bildsystem nach einem der vorhergehenden Ansprüche, das ferner ein Rechenmodul (5) umfasst, das mit einem elektronischen Modul (3) verbunden ist, mit einem Bildschirm oder einer Anzeige, um anatomische und funktionelle Bilder zu zeigen.

**5.** Duales Bildsystem nach einem der vorhergehenden Ansprüche, das zwei oder mehr Compton-Detektoren (2) umfasst.

**6.** Duales Bildsystem nach einem der vorhergehenden Ansprüche, wobei der/die Compton-Detektor(en) (2) neben der Ultraschallbildgebungsvorrichtung (1) an den Seiten der Ultraschallbildgebungsvorrichtung (1) angeordnet ist/sind.

**7.** Duales Bildsystem nach einem der vorhergehenden Ansprüche, wobei die Ultraschallbildgebungsvorrichtung (1) für ihre unabhängige Verwendung von dem/den Compton-Detektor(en) (2) abnehmbar ist.

**8.** Duales Bildsystem nach einem der vorhergehenden Ansprüche, wobei die Datenübertragung zwischen dem/den Compton-Detektor/en (2), dem elektronischen Modul (3) und dem Rechenmodul (5) durch Verkabelung oder durch drahtlose Kommunikation erfolgt.

**9.** Duales Bildsystem nach einem der vorhergehenden Ansprüche, das ferner einen mit einem Compton-Detektor (2) gekoppelten Kollimator umfasst.

**10.** Duales Bildsystem nach einem der vorhergehenden Ansprüche, wobei der relative Abstand und der relative Winkel zwischen jedem Gammaerfassungsbereich jedes Compton-Detektors (2) mechanisch einstellbar sein können.

**11.** Duales Bildsystem nach einem der vorhergehenden Ansprüche, wobei mindestens ein Compton-Detektor (2) zwei oder mehr Gammastrahlen-Erfassungsbereiche umfasst, die im Wesentlichen in der gleichen Erfassungsrichtung ausgerichtet sind und deren Erfassungsoberflächen im Wesentlichen parallel zueinander sind.

**12.** Duales Bildsystem nach einem der vorhergehenden Ansprüche, das eine Nadel umfasst, um geführte Biopsien durchzuführen.

**13.** Verfahren zum Erhalten dualer anatomischer und funktioneller Bilder einer Versuchsperson, das die Verwendung eines dualen Bildsystems nach einem der vorhergehenden Ansprüche 1-12 und das Ausführen der folgenden Schritte umfasst:

- das System wird auf einer Oberfläche oder dem Körper einer Versuchsperson eingesetzt;
- anatomische Informationen der Oberfläche oder des Körpers werden von der Ultraschallbildgebungsvorrichtung (1) erzeugt;
- funktionelle Informationen der Oberfläche oder des Körpers werden durch den/die Compton-Detektor(en) (2) erzeugt;
- die von der Ultraschallbildgebungsvorrichtung (1) und dem/den Compton-Detektor(en) (2) erhaltenen Signale werden vom elektronischen Modul (3) verarbeitet;
- die verarbeiteten Signale werden an das Rechenmodul (5) gesendet;
- die anatomischen und funktionellen Bilder werden in Echtzeit auf einer Anzeige von dem Rechenmodul (5) angezeigt.

**Revendications**

**1.** Système d'image double, convenant pour une utilisation dans des diagnostics oncologiques et des biopsies guidées en temps réel, qui comprend :

- un dispositif d'imagerie à ultrasons (1) pour obtenir des signaux associés à une ou plusieurs images anatomiques ;
- un détecteur de rayons gamma (2) pour obtenir des signaux associés à une ou plusieurs images fonctionnelles ;
- un module électronique (3) pour traiter des signaux obtenus par le dispositif d'imagerie à ultrasons (1) et par le détecteur de rayons gamma (2) ;
- un boîtier (4) logeant un dispositif d'imagerie à ultrasons (1) et un détecteur de rayons gamma (2) ;

et **caractérisé en ce que :**

- le détecteur de rayons gamma (2) est un détecteur de diffusion Compton des rayons gamma, comprenant au moins deux régions de détection de rayons gamma, sensiblement alignées dans la même direction de détection ;
- le dispositif d'imagerie à ultrasons (1) et le détecteur de rayons gamma (2) comprennent des surfaces de détection formant un angle compris entre 0 et 90 degrés ;
- les régions de détection de rayons gamma du détecteur de diffusion Compton comprennent au moins deux détecteurs de rayons gamma individuels (2a, 2b) pourvus d'une pluralité de cristaux à scintillation (6) pour la détection de rayonnement gamma et d'une pluralité de photomultiplicateurs à semiconducteur (7) pour amplifier le signal détecté par lesdits cristaux à scintillation (6).

2. Système d'image double, selon la revendication précédente, dans lequel les cristaux à scintillation (6) comprennent un cristal à scintillation pixélisé ou monolithique, tel que : LaBr$_3$, LaCl$_3$ ou LYSO ; lesdits cristaux à scintillation (6) étant couplés aux photomultiplicateurs (7).

3. Système d'image double, selon la revendication 1, dans lequel le détecteur Compton (2) comprend un seul volume de détection, capable de faire la distinction entre deux interactions ou plus du même rayon gamma à l'intérieur dudit volume.

4. Système d'image double, selon l'une quelconque des revendications précédentes, qui comprend en outre un module de calcul (5) connecté à un module électronique (3), avec un écran ou un affichage afin de visualiser des images anatomiques et fonctionnelles.

5. Système d'image double, selon l'une quelconque des revendications précédentes, qui comprend deux détecteurs Compton (2) ou plus.

6. Système d'image double, selon l'une quelconque des revendications précédentes, dans lequel le ou les détecteurs Compton (2) sont disposés en juxtaposition avec le dispositif d'imagerie à ultrasons (1) sur les côtés dudit dispositif d'imagerie à ultrasons (1).

7. Système d'image double, selon l'une quelconque des revendications précédentes, dans lequel le dispositif d'imagerie à ultrasons (1) est détachable pour son utilisation indépendante du ou des détecteurs Compton (2).

8. Système d'image double, selon l'une quelconque des revendications précédentes, dans lequel la transmission de données entre le ou les détecteurs Compton (2), le module électronique (3) et le module de calcul (5) est réalisée par câblage ou par communication sans fil.

9. Système d'image double, selon l'une quelconque des revendications précédentes, qui comprend en outre un collimateur couplé à un détecteur Compton (2).

10. Système d'image double, selon l'une quelconque des revendications précédentes, dans lequel la distance relative et l'angle relatif entre chaque région de détection gamma de chaque détecteur Compton (2) peuvent être ajustables mécaniquement.

11. Système d'image double, selon l'une quelconque des revendications précédentes, dans lequel au moins un détecteur Compton (2) comprend deux régions de détection de rayons gamma ou plus, sensiblement alignées dans la même direction de détection et avec des surfaces de détection sensiblement parallèles les unes aux autres.

12. Système d'image double, selon l'une quelconque des revendications précédentes, qui comprend une aiguille pour effectuer des biopsies guidées.

13. Procédé pour obtenir des images anatomiques et fonctionnelles doubles d'un sujet qui comprend l'utilisation d'un système d'image double selon l'une quelconque des revendications 1 à 12 précédentes et la mise en oeuvre des étapes suivantes :

- le système est déployé sur une surface ou le corps d'un sujet ;
- des informations anatomiques de ladite surface ou dudit corps sont générées par le dispositif d'imagerie à ultrasons (1) ;

- des informations fonctionnelles de ladite surface ou dudit corps sont générées par le ou les détecteurs Compton (2) ;
- les signaux obtenus par le dispositif d'imagerie à ultrasons (1) et le ou les détecteurs Compton (2) sont traités par le module électronique (3) ;
- les signaux traités sont envoyés au module de calcul (5) ;
- les images anatomiques et fonctionnelles sont visualisées en temps réel sur un afficheur à partir du module de calcul (5).

**FIG. 1**

**FIG. 2**

FIG.3

FIG.4

(A)

(B)

FIG.5

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005013291 A